# EUROPÄISCHE PATENTANMELDUNG

(11) **EP 1 774 947 A1**
(43) Veröffentlichungstag der Anmeldung: **18.04.2007**
(21) Anmeldenummer: 06006763.4
(22) Anmeldetag: 30.03.2006
(51) Int. Cl.: A61H 1/00

(54) **Massagemöbel**

(30) Priorität: 12.10.2005 DE 202005016015 U
(71) Anmelder: Health Med GmbH, 87656 Untergermaringen (DE)
(72) Erfinder: Englert, Frank, 87662 Kaltental-Blonhofen (DE)
(74) Vertreter: Heinze, Ekkehard

(57) **Zusammenfassung**

Massagemöbel, insbesondere Massagematratze, -liege oder -sessel, mit einer aktiven oder passiven Massageeinrichtung, mit einer integrierten Beleuchtungseinrichtung zur Emission von Licht in mindestens einer vorbestimmten Farbe und/oder einer Aromatherapie-Einrichtung zur Freisetzung eines in einem Duftmittel-Reservoir aufbewahrten Duftmittels in Gas- oder Nebelform mit therapeutischer Wirkung.

## Beschreibung

Die Erfindung betrifft ein Massagemöbel mit einer aktiven oder passiven Massageeinrichtung, welches insbesondere als Massagematratze bzw. -liege oder als Massagesessel ausgeführt sein kann.

Derartige Massagemöbel sind in vielgestaltigen Ausführungen bekannt und im praktischen Einsatz. So ist eine Massageliege, die einen Schaumstoffkörper und in diesen eingesetzte Vibrationsmotoren aufweist, aus der DE 295 07 712 U1 bekannt.

Passiv wirkende, also ohne motorischen Antrieb auskommende und ihre Massagewirkung aus den Körperbewegungen des Nutzers beziehende Massagemöbel sind etwa aus der EP 0 585 588 B1 oder US 2005/0015031 A1 bekannt.

Aus diesen und weiteren Druckschriften ist es auch bekannt, mindestens einen Abschnitt der Liege- oder Sitzfläche eines derartigen Massagemöbels mit Noppen oder ähnlichen Vorsprüngen zur Intensivierung der Massagewirkung zu versehen.

Auch bereits aus der US 2,800,897 und der US 2,850,009 ist ein Massagekissen mit einem aktiven Schwingungserzeuger bekannt, welches außerdem eine. Heizeinrichtung zur Erwärmung der Liegefläche aufweist. Bekannt ist es - etwa aus dem DE-GM 78 24 675 - auch, zur Temperierung von Massageliegen eine elektrisch beheizbare Matte bereitzustellen, die bevorzugt ein Dunkelstrahlelement ohne Heizdrähte aufweist.

In der JP 2000-279475 wird ein Massagesessel beschrieben, der eine im Kopf- und Rückenteil verschiebliche IR-Bestrahlungseinrichtung enthält und dem Nutzer neben der Massage eine IR-Bestrahlung/Thermotherapie bietet.

Aus der EP 0 774 246 A2 oder DE 20 2004 002 685 U1 sind multifunktionale Massagevorrichtungen bekannt, welche jeweils insbesondere Matratzen- bzw. Mattenform haben und neben Mitteln zur Erzeugung eines Massageeffektes auch solche zur Infrarot- und Magnetbehandlung des Körpers aufweisen. In der letztgenannten Druckschrift wird auch ein Kopfhörer und/oder brillenartiges Sehgerät als Zusatzgerät beschrieben, welches gemeinsam mit den übrigen Therapiemitteln über ein separates Bedienteil steuerbar sind. Funktion bzw. therapeutische Wirkung des Kopfhörers und Sehgerätes sind hier nicht genauer beschrieben.

Der Erfindung liegt die Aufgabe der Bereitstellung eines verbesserten Massagemöbels der gattungsgemäßen Art zugrunde, welches dem Nutzer insbesondere erweiterte Therapie- und Entspannungsmöglichkeiten bietet und somit einen erheblichen Mehrwert gegenüber bekannten Massagemöbeln der oben skizzierten Art hat.

Diese Aufgabe wird durch ein Massagemöbel mit den Merkmalen des Anspruchs 1 gelöst. Zweckmäßige Fortbildungen des Erfindungsgedankens sind Gegenstand der abhängigen Ansprüche.

Die Erfindung schließt die wesentliche Überlegung ein, in einem Massagemöbel neben bekannten therapeutischen Funktionen eines solchen Möbels auch die Möglichkeit einer Licht- und/oder Aromatherapie integriert bereitzustellen. Sie schließt weiter den Gedanken ein, hierzu einerseits eine integrierte Beleuchtungsanlage zur Emission von Licht in mindestens einer vorbestimmten Farbe vorzusehen. Alternativ hierzu oder in Kombination hiermit ist eine Aromatherapie-Einrichtung zur Freisetzung eines in einem Duftmittel-Reservoir aufbewahrten Duftmittels in therapeutisch wirksamer Form (Dampf oder Nebel) vorgesehen.

Eine bevorzugte Ausführung der Erfindung zeichnet sich aus durch eine Heizeinrichtung zur Erwärmung mindestens eines Abschnitts einer Liege- oder Sitzfläche und/oder eines Heizabschnittes der Aromatherapie-Einrichtung. Vorteilhafterweise ist hier vorgesehen, dass die Heizeinrichtung eine IR-Lampe oder eine breitbandig im IR-Bereich und sichtbaren Bereich emittierende Lampe aufweist. In einer besonders zweckmäßigen Ausführung kann die breitbandig emittierende Lampe zugleich die Beleuchtungseinrichtung als Mittel für eine Lichttherapie oder einen Teil derselben ausbilden.

Eine wegen ihrer Variabilität besonders vorteilhafte weitere Ausführung des Massagemöbels zeichnet sich dadurch aus, dass die oder mindestens eine Beleuchtungseinrichtung ein Farbfilter zur Vorbestimmung der Lichtfarbe aufweist, wobei insbesondere das oder mindestens ein Farbfilter wechselbar ist. Hierbei kann sinnvollerweise die oder eine Beleuchtungseinrichtung oder ein dieser zugeordneter Reflektor so angeordnet sein, dass diese(r) im Gebrauch des Massagemöbels im Wahrnehmungsbereich des Nutzers liegt. Alternativ hierzu oder in Kombination hiermit ist bevorzugt vorgesehen, dass die Beleuchtungseinrichtung mehrere in einer Liege- oder Sitzfläche verteilte Lampen aufweist, die Licht der gleichen Farbe oder verschiedener Farben emittieren.

Mit den vorstehend erwähnten Ausführungen ist eine Lichttherapie in Verbindung mit einer Massage und wahlweise Wärme- bzw. Aromatherapie anzuwenden - die Lichttherapie kann aber auch eigenständig genutzt werden. Der Einsatz verschiedener Farben bei der Lichttherapie ist vorgesehen, wobei die Farben alternativ oder in Kombination miteinander eingesetzt werden, um eine farb-spezifische therapeutische Wirkung zu erreichen. Je nach Wahl der Lichtfarbe - vorteilhaft zu bestimmen über ein entsprechend gefertigtes Farbfilter - kann die Wirkung von beruhigend über entspannend und Wohlgefühl erzeugend bis hin zu belebend und aktivierend reichen.

Da die Lichttherapie sich vor allem auf die Haut und das darunterliegende Gewebe auswirken, aber auch über das Sehvermögen wirken kann, ist bei verschiedenen Varianten der Erfindung die Auswirkung auf diese verschiedenen Organe des Körpers vorgesehen. Hiermit können u.a. Hautkrankheiten und Entzündungen im Körper, aber auch nervliche und psychische Erkrankungen behandelt werden. Der bevorzugt vorgesehene Austausch von Filtern zur Realisierung unterschiedlicher Farbtöne ermöglicht in einfacher Weise die Abstimmung auf einen spezifischen Therapie- oder Wellness-Effekt.

In einer weiteren bevorzugten Ausgestaltung des Erfindungsgedanken ist vorgesehen, dass die Aromatherapie-Einrichtung mehrere in eine Liege- oder Sitzfläche verteilte Duftmittel-Emitter aufweist. Sofern mehrere Duftmittel-Emitter integriert sind, sollten zur Erleichterung der Handhabung insbesondere mindestens einige davon ein gemeinsames Duftmittel-Reservoir haben. Ähnlich wie bei der Ausführung der Lichttherapie-Mittel ist auch hier einer weiteren Ausgestaltung vorgesehen, dass die Aromatherapie-Einrichtung einen Duftmittel-Emitter zur Freisetzung von Duftmitteln aufweist, der im Gebrauch des Massagemöbels in Kopfnähe des Nutzers platziert ist.

Die vorgesehenen Mittel zur Aromatherapie machen das Massagemöbel geeignet als Heilmittel z.B. bei Infekten oder Atemwegserkrankungen. Des weiteren wirkt sich die Aromatherapie positiv auf das gesamte Immunsystem aus, da die therapeutisch wirksamen Duftmittel nicht nur über die Atmung aufgenommen werden, sondern auch über die Haut. Hiermit ist also auch die Behandlung von bestimmten Hautkrankheiten möglich. Auch innere Organe, wie die Lunge, die Nieren, das Herz etc. können in ihrer Funktion .stabilisiert oder verbessert werden, indem über die Atmungsorgane und/oder die Haut gas- bzw. dampfförmig bereitgestellte Wirkstoffe aufgenommen werden.

Eine in sinnvoller Weise Aufwand und damit Kosten sparende Variante des vorgeschlagenen Massagemöbels zeichnet sich dadurch aus, dass mindestens einigen der Duftmittel-Emitter jeweils direkt eine Heizeinrichtung, insbesondere eine IR-Lampe oder breitbandige Lampe, zugeordnet ist. Die Wärmestrahlung dieser Lampe erwärmt einerseits das Duftmittel und führt zu dessen Verdunstung und damit zur Bildung eines Duftmittel-Dampfes oder -Nebels über dem Massagemöbel, andererseits wird dadurch mindestens ein Abschnitt der Liege- oder Sitzfläche in für den Benutzer angenehmer und thermotherapeutisch wirksamer Weise erwärmt.

In einer weiteren Ausgestaltung ist vorgesehen, dass mindestens ein Teil der Duftmittel-Emitter durch Oberflächenabschnitte mindestens eines Duftmittel-Reservoirs gebildet ist. Konstruktiv einfach und sicher und bequem im Gebrauch ist eine Ausführung, bei der der oder jeder Duftmittel-Emitter eine mindestens von innen nach außen gas- oder nebel-durchlässige, aber flüssigkeits-undurchlässige Öffnung, bevorzugt eine Vielzahl derartiger Öffnungen, aufweist. Hierbei kann zumindest ein Teil der Öffnungen in der Liege- oder Sitzfläche angeordnet sein. Alternativ hierzu oder in Kombination hiermit ist weiterhin vorgesehen, dass zumindest ein Teil der Öffnungen seitlich an der Liege- oder Sitzfläche angeordnet ist.

In Weiterverfolgung des oben erwähnten Kombinationseffektes einer IR-Lampe oder breitbandigen Lampe als wärmeerzeugendes Element und zugleich Heizabschnitt eines Duftmittel-Emitters ist bevorzugt weiter vorgesehen, dass die IR-Lampe oder breitbandige Lampe, bezogen auf die Liege- bzw. Sitzfläche, unterhalb des Duftmittel-Reservoirs angeordnet ist. Dessen Wandung ist für die Strahlung der IR-Lampe oder breitbandige Lampe mindestens teildurchlässig derart, dass mindestens ein Teil der Strahlung aus der Liege- bzw. Sitzfläche emittiert wird. Hierbei durchsetzt die IR- oder breitbandige Strahlung also das Duftmittel-Reservoir und trifft nach dessen Passieren auf den Körper des Benutzers auf.

Hierbei lässt sich im übrigen durch geeignete Farbgebung der Wandung des Duftmittel-Reservoirs zugleich der Farbton des sichtbaren Anteils einer breitbandigen Strahlung auf einfache und kostengünstige Weise (ohne gesondertes Farbfilter) einstellen. Zudem ermöglicht die Wahl eines geeigneten Wandungsmaterial und einer geeigneten Wandungsdicke auch die Vorgabe eines gewünschten Verhältnisses zwischen den für die Freisetzung des Duftmittels wirksamen und den zum Körper des Benutzers gelangenden Anteilen der Strahlung.

In einer weiteren bevorzugten Ausgestaltung der Erfindung ist vorgesehen, dass das oder jedes Duftmittel-Reservoir über eine Befülleinrichtung auffüllbar ist. Es versteht sich, dass alternativ auch Einweg-Duftmittelbehälter zum Einsetzen in dazu vorgesehene Vertiefungen oder Hohlräume des Massagemöbels vorgesehen sein können, die nach Entleerung ausgetauscht werden. Bei der wieder-befüllbaren Variante sieht eine vorteilhafte Ausgestaltung vor, dass die Befülleinrichtung einen Schlauchanschluss aufweist. Alternativ hierzu kann die Befülleinrichtung einen öffenbaren Oberflächenabschnitt der Liege- oder Sitzfläche aufweisen. Die letztgenannte Variante kann beispielsweise mit einem die Öffnung des Duftmittel-Reservoirs umgebenden wiederverschließbaren Klebstoffstreifen o.ä..realisiert sein.

Eine wesentliche Erweiterung der Verwendungsmöglichkeiten des vorgeschlagenen Massagemöbels lässt sich in einer weiteren bevorzugten Ausführung erreichen, bei der die oder mindestens eine Aromatherapie-Einrichtung und/oder Beleuchtungseinrichtung in einem lösbaren Teil des Massagemöbels angeordnet ist. Noch universeller wird die Einsatzbarkeit, wenn das lösbare Teil auch die oder mindestens eine Heizeinrichtung aufweist und/oder das lösbare Teil auch die oder eine aktive Massageeinrichtung aufweist. Die genaue konstruktive Ausgestaltung lässt sich dabei auf den angesprochenen Nutzerkreis ebenso zuschneiden wie auf das angezielte Preissegment. So kann es sinnvoll sein, die Mittel für die Licht- und/oder Aromatherapie trennbar von den Massagemitteln vorzusehen, wobei letztere im eigentlichen Grundkörper des Massagemöbels verbleiben. Das abtrennbare Teil kann dann als*eigenständige Lichttherapie-Anlage oder unabhängiger Inhalator eingesetzt werden und relativ kompakt und leicht ausgeführt sein. Andererseits ist es auch möglich, alle oder nahezu alle Therapieeinrichtungen in einem vom eigentlichen Möbel lösbaren universellen Therapieelement zu konzentrieren und dennoch, nach Wahl des Nutzers, einzeln oder miteinander kombiniert in Betrieb zu nehmen.

Im Hinblick auf die hervorgehobene Bedeutung der Atmungsorgane für die Wirkung einer Aromatherapie sowie der Augen für die Wirkung einer Lichttherapie ist es von Vorteil, wenn das erwähnte lösbare Teil als Kopfpolster bzw. -kissen ausgestaltet ist, es sind aber auch Ausführungen beispielsweise als lösbare Rückenpolster oder Seitenteile o.ä. möglich.

Weitere vorteilhafte Ausführungen der Erfindung betreffen die Steuerung der verschiedenen Therapiemittel im Massagemöbel: Vorgesehen ist hierzu insbesondere eine Programmsteuereinrichtung zur Zeit- und/oder Intensitätssteuerung der Beleuchtungseinrichtung und/oder Aromatherapie-Einrichtung, bevorzugt beider Einrichtungen. Eine bevorzugte Ausführung zeichnet sich dadurch aus, dass die Programmsteuereinrichtung eine Mehrzahl von Kanälen zur separaten Steuerung mindestens von Gruppen mehrerer Beleuchtungseinrichtungen und/oder Aromatherapie-Einrichtungen oder der einzelnen Einrichtungen aufweist.

Es versteht sich, dass eine flexible Steuerung auch die selektive Zu- und Abschaltung einzelner Therapiemittel umfasst - wahlweise mit oder ohne gleichzeitigem Betrieb von Massageelementen mit den Wärme-, Licht- und Aromatherapie-Einrichtungen. Die Programmsteuerung kann hierbei weitgehend manuell, mit Unterstützung durch Timer und flexibel kombinierbare Stufen- oder Gradienten-Programme, erfolgen. Es können aber in der Programmsteuereinheit auch vorbestimmte Therapieprogramme gespeichert sein, in denen Zeitpunkt, Zeitdauern und Intensitäten des Betriebes der verschiedenen verfügbaren Therapieeinrichtungen definiert abgelegt sind.

In einer besonders einfach und bequem handhabbaren Ausführung weist die Programmsteuereinheit ein Handsteuerteil auf, das insbesondere als drahtlose Fernbedienung des Massagemöbels ausgebildet ist. Es versteht sich, dass das Handsteuerteil auch als integriertes Bedienteil ausgeführt oder über ein Steuerkabel mit dem Massagemöbel verbunden sein kann, ein Fernbedienteil auf Basis einer drahtlosen Verbindung (etwa IR oder Bluetooth) kann aber Gebrauchswertvorteile bieten und zu einer wertigeren Anmutung des Massagemöbels beitragen.

Vorteile und Zweckmäßigkeiten der Erfindung werden deutlicher anhand der nachfolgenden Beschreibung einiger ausgewählter Ausführungsbeispiele anhand der Figuren. Von diesen zeigen:
- Fig. 1: eine skizzenartige perspektivische Darstellung einer Massagematratze gemäß einer ersten Ausführungsform der Erfindung,

- Fig. 2: eine Detaildarstellung hierzu,
- Fig. 3: eine skizzenhafte perspektivische Darstellung einer Massageliege gemäß einer zweiten Ausführungsform der Erfindung sowie
- Fig. 4: eine skizzenhafte perspektivische Darstellung eines Massagesessels gemäß einer dritten Ausführungsform der Erfindung.

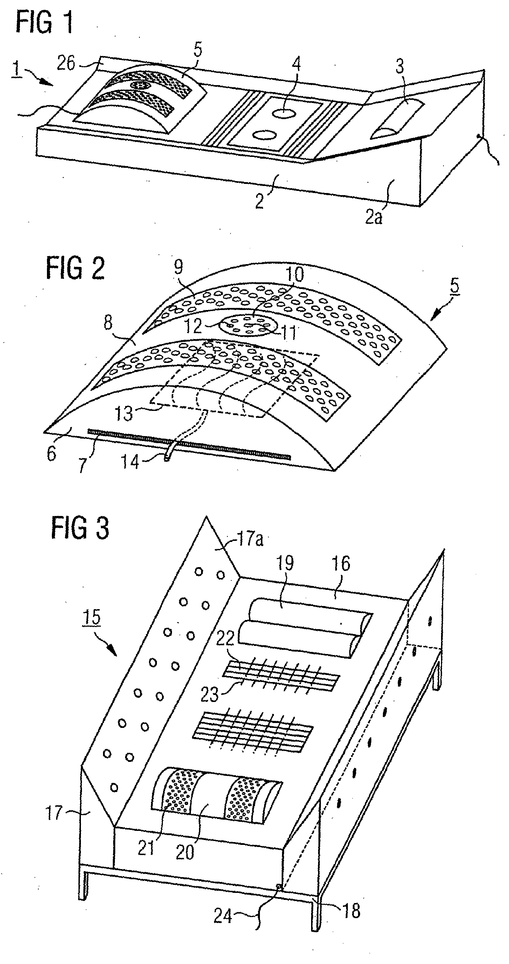

Fig. 1 zeigt eine Massagematratze 1 mit in üblicher Weise eingebauten (hier nicht dargestellten) motorischen Vibrations-Massageelementen in einem Schaumstoff- bzw. Latex-Grundkörper 2, der einen keilförmig ansteigenden Kopfbereich 2a aufweist. Zudem hat der Matratzen-Grundkörper 2 eingearbeitete Seitenwülste 2b zur besseren seitlichen Führung des Benutzers. Auf dem Kopfabschnitt 2a ist ein - bevorzugt verschieblich befestigbares - Nackenkissen 3 aufgesetzt. Im Rückenbereich sind zwei Infrarotlampen 4 als Wärmetherapie-Einrichtungen eingelassen. Im Unterschenkel-/Fußbereich ist auf den Grundkörper 2 ein konvex gewölbtes Fußkissen 5 (bevorzugt ebenfalls verschieblich, etwa mittels Klettstreifen) aufgesetzt, welches im Detail in Fig. 2 gezeigt ist.

Fig. 2 zeigt, dass das Fußkissen 5 ebenfalls einen Schaumstoff-Grundkörper 6 aufweist, der einen mit einem Reißverschluss 7 versehenen Bezug 8 hat. Auf den Bezug 8 sind in den seitlichen Oberflächenbereichen zwei langgestreckte Noppen-Flächen 9 zur Verbesserung der Massagewirkung aufgebracht. Zwischen diesen ist in den Grundkörper 6 eine Beleuchtungs-Einrichtung 10 eingearbeitet, die eine zentral angeordnete hellere Lampe 11 und mehrere um diese herum positionierte schwächere Leuchtelementen (ggf. LEDs) 12 mit einer anderen Lichtfarbe als derjenigen der zentralen Lichtquelle 11 aufweist.

Im Inneren des Grundkörpers 6 befindet sich ein Duftmittel-Reservoir 13, das durch einen flexiblen Befüllstutzen 14 mit einem flüssigen Duftmittel befüllt werden kann. Im Bereich des Beleuchtungseinrichtung 10 ist dem Duftmittel-Reservoir 13 ein (hier nicht dargestellter) Duftmittel-Emitter zugeordnet, über den bei Zimmertemperatur verdunstendes Duftmittel in die Atmosphäre über der Massagematratze 1 gelangt und für den Benutzer im Sinne einer Aromatherapie wirksam werden kann.

Der Duftmittel-Emitter kann beispielsweise einen dampfdurchlässigen Oberflächenbereich des Duftmittel-Reservoirs 13 aufweisen, zu dessen Lage im Grundkörper 6 ein ebenfalls in besonderem Maße dampfdurchlässiger Bereich des Bezuges 8 korrespondiert. Der Bezug kann aber auch insgesamt dampfdurchlässig ausgeführt sein.

Fig. 3 als weitere Ausführungsform der Erfindung eine Massageliege 15, die aus einem Matratzengrundkörper 16 und zwei Liegen-Seitenteilen 17 zu beiden Seiten des Grundkörpers 16 auf einem gemeinsamen Gestell 18 besteht. Die Seitenteile 17 können mit der Matratze 16 beispielsweise über Klettverschlüsse lösbar verbunden sein, so dass verschiedenartig ausgeformte Seitenteile mit der Matratze zur Realisierung unterschiedlicher Liegen-Konfigurationen verbindbar sind.

Ähnlich wie die Massagematratze 1 nach Fig. 1, hat die Matratze 16 ein separat ausgeformtes Nackenkissen 19 und ein ausgeformtes Fußteil 20 mit zwei genoppten Oberflächenabschnitten 21. Zudem sind im Becken- und Oberschenkelbereich der Matratze 16 zwei Heizeinrichtungen 22 mit räumlich jeweils zugeordnetem Duftstoff-Emitter 23 vorgesehen. Die Duftstoff-Emitter 23 sind hier lediglich symbolisch als reguläre Anordnungen strichpunktierter Linien dargestellt, die die Dampfaustrittsöffnungen eines (hier nicht dargestellten) Duftstoff-Reservoirs darstellen sollen. Die jeweils zugeordnete Heizeinrichtung 22 liegt unterhalb des Duftstoff-Reservoirs und erwärmt dessen Inhalt zur Beförderung der Verdunstung des Aromatherapeutikums, zugleich aber auch den Becken- bzw. Oberschenkelbereich der Matratzenoberfläche.

In der nach innen zur Matratze 16 gewandten, über diese überstehenden Seitenfläche 17a der Seitenteile 17 sind jeweils eine Vielzahl von Leuchtmitteln (LEDs oder Kleinlampen) angeordnet, die von vornherein als Emitter für verschiedene Lichtfarben ausgewählt oder alternativ vom gleichen Typ, aber mit unterschiedlichen Farbfiltern bestückt sein können. Bei der hier dargestellten Ausführung einer Massageliege benötigen sowohl das mittlere Matratzen-Teil als auch die Seitenteile einen Stromanschluss; dieser kann jedoch zur Vermeidung von "Kabelsalat" in der Wohnung des Benutzers durch einen zentralen Netzanschluss 24 an der Massagematratze 16 und (nicht dargestellte) Steckverbindungen zwischen dieser und den Seitenteilen realisiert sein.

Alternativ zur hier dargestellten Ausführung können auch im mittleren Matratzen-Teil Beleuchtungseinrichtungen für die Lichttherapie und in den Seitenteilen Duftstoff-Emitter für die Aromatherapie (insbesondere im Kopfbereich des Benutzers) angeordnet sein.

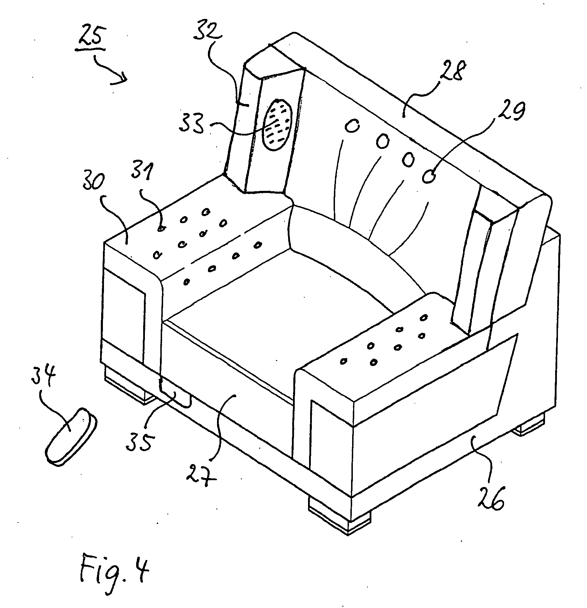

Fig. 4 zeigt einen Massagesessel 25, bei dem in einem stabilen Grundgestell 26 mehrere flexibel kombinierbare Komponenten zur Realisierung verschiedener Therapiefunktionen aufgenommen sind. Hierzu zählen zunächst ein zentrales Sitzkissen 27 mit eingebauten (nicht dargestellten) Vibrations-Massageeinrichtungen, ein Rückenkissen 28 mit einer Mehrzahl von Infrarotlampen 29 zur Wärmetherapie - welches ebenfalls eingebaute Vibrations-Massageeinrichtungen enthalten kann -, zwei im Querschnitt annähernd L-förmige Armstützen 30 mit einer Mehrzahl von Beleuchtungseinrichtungen 31 zur Lichttherapie und zwei Ohrenpolster 32, die jeweils einen Duftstoff-Emitter 33 auf der zum Benutzer gewandten Oberfläche aufweisen.

Daneben gehört zum Massagesessel 25 eine drahtlose Fernbedienung 34, die über eine Bluetooth-Verbindung mit einem Empfangsteil 35 im Grundgestell 26 in Verbindung steht, welches Steuermittel zur Ansteuerung der verschiedenen Therapieeinrichtungen umfasst und mit diesen signalmäßig verbunden ist.

Es versteht sich für den Fachmann, dass bei einem solchen Aufbau des Massagesessels die Zuordnung verschiedener Therapieeinrichtungen zu den einzelnen Komponenten - Sitzkissen, Rückenkissen, Armstütze und Ohrpolster o.ä. - vielfältig variiert werden kann und durch Bereitstellung unterschiedlich komplexer Funktionen sich ein breites Bedarfsspektrum von Low-Cost- bis hin zu High-End-Lösungen realisieren lässt.

Auch im übrigen ist die Ausführung der Erfindung nicht auf die oben beschriebenen Beispiele und hervorgehobenen Aspekte beschränkt, sondern lediglich durch den Schutzbereich der anhängenden Schutzansprüche.

## Patentansprüche

1. Massagemöbel, insbesondere Massagematratze, -liege oder -sessel, mit einer aktiven oder passiven Massageeinrichtung,
**gekennzeichnet durch**
eine integrierte Beleuchtungseinrichtung zur Emission von Licht in mindestens einer vorbestimmten Farbe und/oder
eine Aromatherapie-Einrichtung zur Freisetzung eines in einem Duftmittel-Reservoir aufbewahrten Duftmittels in Gas- oder Nebelform mit therapeutischer Wirkung.

2. Massagemöbel nach Anspruch 1, **gekennzeichnet durch**
eine Heizeinrichtung zur Erwärmung mindestens eines Abschnitts einer Liege- oder Sitzfläche und/oder eines Heizabschnittes der Aromatherapie-Einrichtung.

3. Massagemöbel nach Anspruch 2, **dadurch gekennzeichnet, dass**
die Heizeinrichtung eine IR-Lampe oder eine breitbandig im IR-Bereich und sichtbaren Bereich emittierende Lampe aufweist.

4. Massagemöbel nach Anspruch 3, **dadurch gekennzeichnet, dass**
die breitbandig emittierende Lampe zugleich die Beleuchtungseinrichtung oder einen Teil derselben ausbildet.

5. Massagemöbel nach einem der vorangehenden Ansprüche, **dadurch gekennzeichnet, dass**
die oder mindestens eine Beleuchtungseinrichtung ein Farbfilter zur Vorbestimmung der Lichtfarbe aufweist.

6. Massagemöbel nach Anspruch 5, **dadurch gekennzeichnet, dass** das oder mindestens ein Farbfilter wechselbar ist.

7. Massagemöbel nach einem der vorangehenden Ansprüche, **dadurch gekennzeichnet, dass**
die oder eine Beleuchtungseinrichtung oder ein dieser zugeordneter Reflektor so angeordnet ist, dass diese(r) im Gebrauch des Massagemöbels im Wahrnehmungsbereich des Nutzers liegt.

8. Massagemöbel nach einem der vorangehenden Ansprüche, **dadurch gekennzeichnet, dass**
die Beleuchtungseinrichtung mehrere in einer Liege- oder Sitzfläche verteilte Lampen aufweist.

9. Massagemöbel nach Anspruch 8, **dadurch gekennzeichnet, dass**
den Lampen jeweils separat ein Farbfilter zugeordnet oder zuordenbar ist.

10. Massagemöbel nach einem der vorangehenden Ansprüche, **dadurch gekennzeichnet, dass**
die Aromatherapie-Einrichtung mehrere in eine Liege- oder Sitzfläche verteilte Duftmittel-Emitter aufweist.

11. Massagemöbel nach Anspruch 10, **dadurch gekennzeichnet, dass**
mindestens einige der Duftmittel-Emitter ein gemeinsames Duftmittel-Reservoir haben.

12. Massagemöbel nach einem der vorangehenden Ansprüche, **dadurch gekennzeichnet, dass**
die Aromatherapie-Einrichtung einen Duftmittel-Emitter zur Freisetzung von Duftmitteln aufweist, der im Gebrauch des Massagemöbels in Kopfnähe des Nutzers platziert ist.

13. Massagemöbel nach einem der Ansprüche 10 bis 12, **dadurch gekennzeichnet, dass**
mindestens einigen der Duftmittel-Emitter jeweils eine separate Heizeinrichtung, insbesondere eine IR-Lampe oder breitbandige Lampe, zugeordnet ist.

14. Massagemöbel nach einem der Ansprüche 10 bis 13, **dadurch gekennzeichnet, dass**
mindestens ein Teil der Duftmittel-Emitter durch Oberflächenabschnitte mindestens eines Duftmittel-Reservoirs gebildet ist.

15. Massagemöbel nach Anspruch 13 und 14, **dadurch gekennzeichnet, dass**
die IR-Lampe oder breitbandige Lampe, bezogen auf die Liege- bzw. Sitzfläche, unterhalb des Duftmittel-Reservoirs angeordnet und dessen Wandung für die Strahlung der IR-Lampe oder breitbandige Lampe mindestens teildurchlässig ist derart, dass mindestens ein Teil der Strahlung aus der Liege- bzw. Sitzfläche emittiert wird.

16. Massagemöbel nach einem der vorangehenden Ansprüche, **dadurch gekennzeichnet, dass**
das oder jedes Duftmittel-Reservoir über eine Befülleinrichtung auffüllbar ist.

17. Massagemöbel nach Anspruch 16, **dadurch gekennzeichnet, dass**
die Befülleinrichtung einen Schlauchanschluss aufweist.

18. Massagemöbel nach Anspruch 16, **dadurch gekennzeichnet, dass**
die Befülleinrichtung einen öffenbaren Oberflächenabschnitt der Liege- oder Sitzfläche aufweist.

19. Massagemöbel nach einem der Ansprüche 10 bis 18, **dadurch gekennzeichnet, dass**
der oder jeder Duftmittel-Emitter eine mindestens von innen nach außen gas- oder nebel-durchlässige, aber flüssigkeits-undurchlässige Öffnung, bevorzugt eine Vielzahl derartiger Öffnungen, aufweist.

20. Massagemöbel nach Anspruch 19, **dadurch gekennzeichnet, dass**
zumindest ein Teil der Öffnungen in der Liege- oder Sitzfläche angeordnet ist.

21. Massagemöbel nach Anspruch 19 oder 20, **dadurch gekennzeichnet, dass** zumindest ein Teil der Öffnungen seitlich an der Liege- oder Sitzfläche angeordnet ist.

22. Massagemöbel nach einem der vorangehenden Ansprüche, **dadurch gekennzeichnet, dass**
die oder mindestens eine Aromatherapie-Einrichtung und/oder Beleuchtungseinrichtung in einem lösbaren Teil des Massagemöbels angeordnet ist.

23. Massagemöbel nach Anspruch 22, **dadurch gekennzeichnet, dass**
das lösbare Teil auch die oder mindestens eine Heizeinrichtung aufweist.

24. Massagemöbel nach Anspruch 22 oder 23, **dadurch gekennzeichnet, dass** das lösbare Teil auch die oder eine aktive Massageeinrichtung aufweist.

25. Massagemöbel nach einem der Ansprüche 22 bis 24, **dadurch gekennzeichnet, dass**
das lösbare Teil als Kissen oder Polster, insbesondere als Kopfpolster, ausgebildet ist.

26. Massagemöbel nach einem der vorangehenden Ansprüche, **gekennzeichnet durch**
eine Programmsteuereinrichtung zur Zeit- und/oder Intensitätssteuerung der Beleuchtungseinrichtung und/oder Aromatherapie-Einrichtung, bevorzugt beider Einrichtungen.

27. Massagemöbel nach Anspruch 26, **dadurch gekennzeichnet, dass**
die Programmsteuereinrichtung eine Mehrzahl von Kanälen zur separaten Steuerung mindestens von Gruppen mehrerer Beleuchtungseinrichtungen und/oder Aromatherapie-Einrichtungen oder der einzelnen Einrichtungen aufweist.

28. Massagemöbel nach Anspruch 26 oder 27, **dadurch gekennzeichnet, dass** die Programmsteuereinrichtung Heizsteuermittel zur Steuerung der Heizdauer und/oder -leistung der oder mindestens einer Heizeinrichtung aufweist.

29. Massagemöbel nach Anspruch 28, **dadurch gekennzeichnet, dass**
die Heizsteuermittel zugleich zur Zeit- und/oder Intensitätssteuerung der oder mindestens einer Aromatherapie-Einrichtung ausgebildet sind.

30. Massagemöbel nach einem der Ansprüche 26 bis 29, **dadurch gekennzeichnet, dass**
die Programmsteuereinheit Massagesteuermittel zur Steuerung der Massagedauer und/oder -intensität der oder mindestens einer Massageeinrichtung aufweist.

31. Massagemöbel nach einem der Ansprüche 26 bis 30, **dadurch gekennzeichnet, dass**
die Programmsteuereinheit ein Handsteuerteil aufweist, das insbesondere als drahtlose Fernbedienung des Massagemöbels ausgebildet ist.
